# EUROPÄISCHE PATENTSCHRIFT

(11) **EP 1 359 176 B1**
(45) Veröffentlichungstag und Bekanntmachung des Hinweises auf die Patenterteilung: **27.08.2008**
(21) Anmeldenummer: 03009026.0
(22) Anmeldetag: 17.04.2003
(51) Int. Cl.: C08G 64/14, C08G 63/64, C08G 63/20, C07C 49/84, C07C 49/83

(54) **Polycarbonate, Polyestercarbonate und Polyester mit speziellen verzweigten Endgruppen**
Polycarbonates, polyestercarbonates and polyesters having specific branched endgroups
Polycarbonates, polyestercarbonates et polyesters ayant des groupes terminaux spécifiques

(30) Priorität: 30.04.2002 DE 10219229
(43) Veröffentlichungstag der Anmeldung: 05.11.2003
(73) Patentinhaber: Bayer MaterialScience AG, 51368 Leverkusen (DE)
(72) Erfinder: Meyer, Alexander, Dr., 47800 Krefeld (DE); Moethrath, Melanie, Dr., 40227 Düsseldorf (DE); Werner Heuer, Helmut, Dr., 47829 Krefeld (DE); Wehrmann, Rolf, Dr. Dr., 47800 Krefeld (DE); Bruder, Friedrich-Karl, Dr., 47802 Krefeld (DE)

(56) Entgegenhaltungen:
- EP-A- 0 347 682
- EP-B- 0 068 014
- WO-A-98/22522
- DE-A- 2 136 828
- DE-A- 2 746 139
- US-A- 4 153 780
- ATSUSHI MORIKAWA, LATSUMICHI ONO: "Preparation of Poly(ether ketone) Dendrons with Graded Structures" MACROMOLECULES, Bd. 32, 1999, Seiten 1062-1068,
- BROSSI A.; TEITEL S.: 'Total synthesis of racemic cherylline' TETRAHEDRON LETTERS Bd. 11, Nr. 6, 1970, Seiten 417 - 419
- LUZ CARDONA M. ET AL: 'Xanthones from Hypericum reflexum' PHYTOCHEMISTRY Bd. 29, Nr. 9, 1990, Seiten 3003 - 3006

## Beschreibung

Die Erfindung betrifft die Verwendung von phenolischen Verbindungen mit verzweigter Struktur als Endgruppen in Polycarbonaten, Polyestercarbonaten und Polyestern sowie Polycarbonate, Polyestercarbonate und Polyester mit verzweigten Endgruppen, Formkörper und Extrudate aus diesen Polymeren, Verfahren zur Herstellung der Polymere sowie der Formkörper und Extrudate.

Zur Herstellung von Polycarbonaten werden häufig monofunktionelle Endgruppen auf Phenolbasis wie z.B. Phenol, 4-Alkylphenole und 4-Cumylphenol eingesetzt (Kunststoff-Handbuch 3; L. Bottenbruch, Hanser, München 1992, S. 127; EP-A 0 353 594).

Es ist nicht bekannt, dass sich diese herkömmlich eingesetzten Endgruppen positiv auf das Fließverhalten und/oder die Nullviskosität bzw. die thermische Stabilität und damit positiv auf die Verarbeitungseigenschaften der entsprechenden Polycarbonate auswirken.

Die Herstellung von Polycarbonaten mit verzweigten Endgruppen ist grundsätzlich bekannt und beispielsweise in EP-A 0 794 209 und JP-A 06 256 499 beschrieben.

Aus den US-A 3 166 606 und 3 173 891 ist beispielweise *p*-Phenylphenol als Kettenabbrecher für Polycarbonate bekannt. Aus US-A 4 330 663 sind Polyestercarbonate bekannt, in welchen 4-Butylbenzoesäurechlorid als Kettenabbrecher eingesetzt wird.

WO-A 00/50488 beschreibt die Verwendung von Di-*tert*.-alkylphenol als Kettenabbrecher.

Aus der japanischen Offenlegungsschrift 57 13 31 49 sind Polycarbonate bekannt, die mit Phenylpropylphenol, Alkylphenolen oder Naphthol als Endgruppen modifiziert sind.

In WO-A 01/05 866 werden Tritylphenol, Cumylphenol, Phenoxyphenol und Pentadecylphenol als Kettenabbrecher für Polycarbonat beschrieben.

Aus EP-A 1 048 684 und WO-A 99/36 458 sind Polycarbonate bekannt, welche beispielsweise mit 4-(1,1,3,3-Tetramethylbutyl)phenol und weiteren verzweigten Alkylphenolen modifiziert sind.

Aus JP-A 06 25 64 99 sind Polycarbonate mit Endgruppen der Strukturen 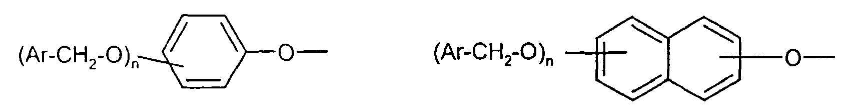 bekannt.

Gemäß DE-A 38 03 939 werden Kettenabbrecher der Formel 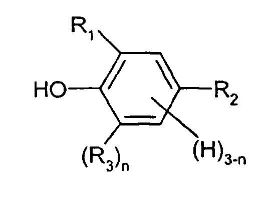 eingesetzt, worin R₁, R₂, R₃ gleich oder verschieden sind und C₂-C₁₂-Alkyl oder C₈-C₂₀-Aralkyl sind, wobei mindestens einer der Reste R₁ oder R₂ ein C₈-C₂₀-Aralkyrest ist, und worin "n" einen Wert zwischen 0,5 und 1 hat.

Aus WO-A 98/22522 sind verzweigte Phenole, deren Verwendung als Endgruppen in Polycarbonaten und Polycarbonate enthaltend solche Endgruppen bekannt. Weiterhin wird als Effekt dieser Endgruppen im Polycarbonat eine geringere Glasübergangstemperatur beschreiben. Es wird jedoch weder etwas über verzweigte Phenole des vorliegend beanspruchten Typs noch über die Auswirkung verzweigter Endgruppen auf die Nullviskosität bzw. thermische Stabilität von Polycarbonaten gesagt.

Die Polyestercarbonate und Polyester mit bekannten Endgruppen besitzen aber den Nachteil einer relativ hohen Nullviskosität und/oder können bei thermischer Belastung zu Molekulargewichtsabbau bzw. Materialverfärbung neigen. So können Polycarbonate, die sekundäre oder tertiäre Wasserstoffatome vor allem in benzylischer Stellung enthalten, unter thermischer Belastung wie z.B. im Extrusionsprozess abgebaut werden, wobei sich das entsprechende Material verfärben kann. Polycarbonate, die Estergruppierungen als Endgruppen enthalten, neigen unter thermischer Belastung zu Umesterungsreaktionen und sind damit für das Schmelzumesterungsverfahren ungeeignet. (Kunststoff-Handbuch, Bd. VIII, S. 150, Carl-Hanser-Verlag, München, 1973).

Ausgehend vom Stand der Technik stellt sich somit die Aufgabe, Polycarbonate, Polyestercarbonate und Polyester, bzw. geeignete phenolische Verbindungen als Endgruppen zu Verfügung zu stellen, welche den Nachteil einer hohen Nullviskosität vermeiden und zugleich zu keinem Abbau unter thermischer Belastung wie z.B. im Extrusionsprozess oder im Spritzguss führen und auch im Schmelzumesterungsverfahren eingesetzt werden können.

Überraschenderweise wurde nun gefunden, dass diese Aufgabe durch den Einsatz von Endgruppen mit spezieller verzweigter, insbesondere dendrimerartiger, Struktur, gelöst wird. Diese Endgruppen beeinflussen die Nullviskosität positiv, d.h. das entsprechende Polycarbonat zeigt bei vergleichbarer Molekulargewichtsverteilung eine niedrigere Nullviskosität und ist damit besser verarbeitbar. Insbesondere haben diese speziellen Endgruppen den Vorteil, dass sie auch bei hohen Temperaturen stabil sind.

Phenolische Endgruppen für Polycarbonat mit dendrimerartiger Struktur auf Basis von Carbonyl- oder Ether-verbrückten Arylsystemen sind bislang nicht bekannt.

Gegenstand der vorliegenden Erfindung sind daher Polycarbonate, Polyestercarbonate und Polyester, die verzweigte, insbesondere dendrimerartige Endgruppen auf Basis von Aryl-CO- bzw. Aryl-O-Verknüpfungen enthalten, die Verwendung solcher Polycarbonate und spezielle, zur Verwendung in den erfindungsgemäßen Polycarbonaten geeignete phenolische Endgruppen bzw. die zugrunde liegenden phenolischen Verbindungen.

Gegenstand der vorliegenden Erfindung sind daher auch die Verwendung der phenolischen Verbindungen gemäß Formel (2) zur Herstellung endgruppenmodifizierter Polymere

Die phenolischen Verbindungen der Formel (2) sind wie folgt definiert: 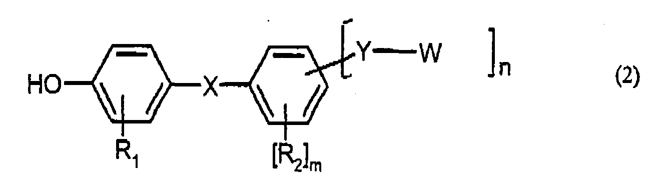 worin
- R₁: für H, lineares oder verzweigtes C₁-C₁₈ Alkyl-, Cl, oder Br,
- X: für eine Einfachbindung oder -0-, oder -CO-,
- R₂: für H, lineares oder verzweigtes C₁-C₁₈ Alkyl-, Cl, oder Br,
- Y: für eine Einfachbindung oder -O-, oder -CO-,
- W: für R⁶ oder einen Rest der Formel 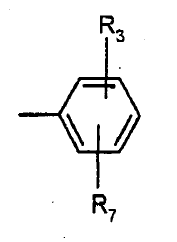
wobei
- R⁶: für lineares oder verzweigtes C₁-C₁₈-Alkyl oder cyclisches C₅-C₁₈-Al-kyl steht,
- R₃, R₇: unabhängig voneinander für H, lineares oder verzweigtes C₁-C₁₈ Al-kyl-, cyclisches C₅-C₁₈ Alkyl-, Phenyl-, Phenyloxy-, Phenylcarboxy-, Benzyl-, Benzyloxy-, Naphthyl- oder Naphthyloxy-, Naphthylcarboxy-reste stehen,
- m: für eine Zahl zwischen 0 und 3 und
- n: für eine Zahl zwischen 2 und 5 stehen,
wobei m und n in Summe ≤ 5 ergibt,

Ganz besonders bevorzugt sind jeweils unabhängig voneinander die phenolischen Verbindungen, welche den Formeln 2a und 3a entsprechen: 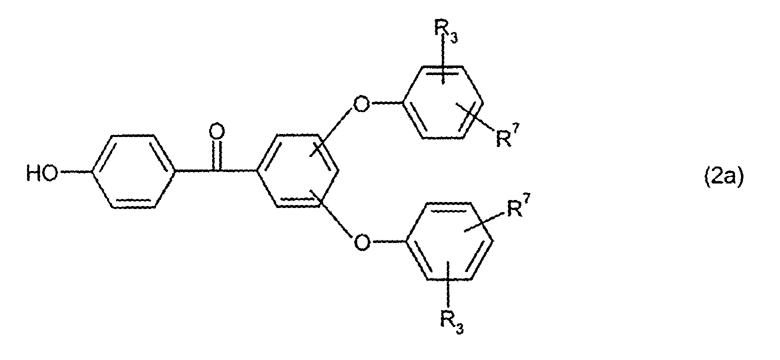 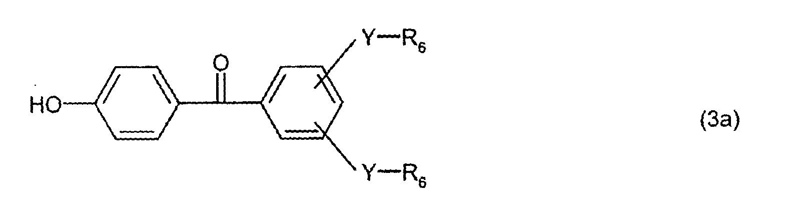 wobei in 2a und 3a die Reste R₃, R₆, R₇ und Y die oben genannten Bedeutungen haben.

Geeignete Endgruppen zur Modifikation von Polycarbonaten, Polyestercarbonaten und Polyestern sind durch Formel (4) dargestellt: 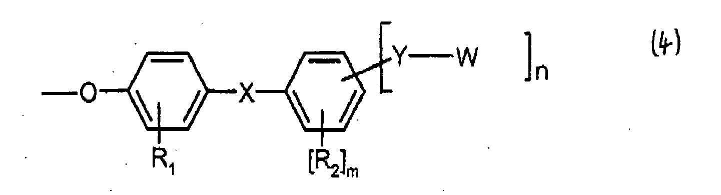 worin
- R₁: für H, lineares oder verzweigtes C₁-C₁₈ Alkyl-, Cl, oder Br,
- X: für eine Einfachbindung oder -O-, oder -CO-,
- R₂: für H, lineares oder verzweigtes C₁-C₁₈ Alkyl-, Cl, oder Br,
- Y: für eine Einfachbindung oder -O-, oder -CO- ,
- W: für R⁶ oder einen Rest der Formel 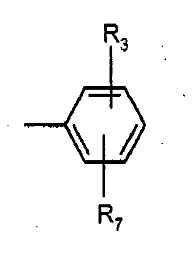
wobei
- R⁶: für lineares oder verzweigtes C₁-C₁₈-Alkyl oder cyclisches C₅-C₁₈-Al-kyl steht,
- R₃, R₇: unabhängig voneinander für H, lineares oder verzweigtes C₁-C₁₈ Alkyl-, cyclisches C₅-C₁₈ Alkyl-, Phenyl-, Phenyloxy-, Phenylcarboxy-, Benzyl-, Benzyloxy-, Naphthyl- oder Naphthyloxy-, Naphthylcarboxy-reste stehen,
- m: für eine Zahl zwischen 0 und 3 und
- n: für eine Zahl zwischen 2 und 5 stehen,
wobei m und n in Summe ≤ 5 ergibt.

Unabhängig voneinander ganz besonders geeignet sind die den phenolischen Verbindungen der Formeln (2a) und (3a) entsprechenden Endgruppen.

Bevorzugt, besonders bevorzugt, ganz besonders bevorzugt oder insbesondere bevorzugt etc. sind Verbindungen, welche die unter bevorzugt, besonders bevorzugt, ganz besonders bevorzugt oder insbesondere bevorzugt etc. genannten Substituenten tragen.

Die oben aufgeführten allgemeinen oder in Vorzugsbereichen aufgeführten Restedefinitionen bzw.. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Gegenstand der vorliegenden Erfindung sind somit auch thermoplastische Polycarbonate, thermoplastische Polyestercarbonate und thermoplastische Polyester mit den phenolischen Verbindungen der Formeln (1), (2) und (3) entsprechenden Endgruppen.

Beispiele für phenolische Verbindungen der Formel (1) sind 3,5-Diphenyloxy-4' hydroxybenzophenon, 3,5-Bis-(*p*-*tert*.-butylphenyloxy)-4'-hydroxybenzophenon, 3,5-Bis-(*p-n*-butylphenyloxy)-4'-hydroxybenzophenon, 3,5-Bis-(3,5-di-*tert*-butylphenyloxy)-4`-hydroxybenzophenon, 3,5-Bis-(*p-iso*-octylphenyloxy)-4'-hydroxybenzophenon, 3,5-Dicyclooctyloxy-4'-hydroxybenzophenon, Dicyclododecyloxy-4' hydroxybenzophenon, 3,5-Bis-(benzoyl)-4'-hydroxybenzophenon und 3,5-Di-tert.-butyl-4'-hydroxybenzophenon.

Verzweigte Fluor-substituierte Hydroxy-oligo(etherketone) sind in der Literatur bekannt (C. J. Hawker, F. Chu, Macromolecules 1996, 29, 4370-4380). Mehrfach-funktionelle Hydroxyverbindungen wie 1,3,5-Tris-(2' hydroxybenzoyl)-benzole sind ebenfalls bekannt (DE 19 59 399).

Die Herstellung der Phenole der Formeln 2 und 3 können nach allgemein bekannten Verfahren erfolgen. So lassen sich z.B. Phenole der Formeln 2 und 3 in denen X und/oder Y für einen Carbonylrest stehen prinzipiell durch Friedel-Crafts-Reaktionen von gegebenenfalls substituierten aliphatischen oder aromatischen Carbonsäurechloriden mit gegebenenfalls substituierten Aromaten herstellen. Verbindungen der Formel 2 und 3 in denen X und/oder Y für ein divalentes Radikal wie -O- steht, lassen sich prinzipiell durch Ullmann-Reaktion von gegebenenfalls substituierten Aromaten mit gegebenenfalls substituierten Phenolaten unter Einwirkung von Kupfer bei Temperaturen zwischen 100 und 230°C herstellen. Alternativ lassen sich diese Diarylether durch Reaktion von gegebenenfalls substituierten Arylboronsäuren mit Phenolaten herstellen. Verbindungen der Formeln 2 und 3, bei denen X und/oder Y für eine Einfachbindung steht lassen sich in bekannter Weise durch C-C-Verknüpfungsreaktionen herstellen. So können diese z.B. durch Ullmann-Reaktion von Halogen-substituierten Aromaten erhalten werden. Vorzugsweise werden Iod-substituierte gegebenenfalls höher substituierte Aromaten unter Einwirkung von Kupfer bei Temperaturen zwischen 100 und 300°C zur Reaktion gebracht. Um die erfindungsgemäßen Phenole der Formeln 2 und 3 zu erhalten, ist es erforderlich in den vorstehend genannten Reaktionen eine geschützte phenolische OH-Gruppe in die jeweilige Verbindung einzuführen, aus der vorzugsweise im letzten Schritt der Synthese die phenolische OH-Gruppe freigesetzt werden kann. Eine solche geschützte phenolische OH-Gruppe kann beispielsweise durch ein gegebenenfalls substituiertes Anisolderivat in den vorstehend beschriebenen Reaktionen in die jeweilige Verbindung eingebracht werden. Zur Freisetzung der phenolischen OH-Gruppe kann die Spaltung des Methylethers beispielsweise mit BBr₃ in Dichlormethan, mit Me₃SiI in Chloroform oder mit wässriger HBr in Essigsäure erfolgen.

Die Herstellung der Phenole der allgemeinen Formel 2a kann nach an sich literaturbekannten Methoden (s. z.B. C. J. Hawker, F. Chu, Macromolecules 1996, 29, 4370-4380, A. Morikawa, K. Ono, Macromolecules 1999, 32, 1062-1068,) erfolgen. So lässt sich Dihalo-benzoesäure mittels gängiger Acylierungsreagenzien wie z.B. Oxalyl- oder Thionylchlorid in das entsprechende Säurechlorid überführen und anschließend mit gegebenenfalls substituierten, allerdings in para-Stellung zur Methoxygruppe unsubstituierten, Anisolderivaten unter Zusatz von Lewis Säuren umsetzen. Bevorzugt lässt sich Difluorbenzoesäure mit Oxalyl- oder Thionylchlorid in das entsprechende Säurechlorid überführen. Dies wird mit gegebenenfalls substituierten, allerdings in para-Stellung zur Methoxygrupope unsubstituierten, Anisolderivaten, bevorzugt unter Einwirkung von z.B. FeCl₃, AlCl₃, BF₃, ZnCl₂, SnCl₄ oder SbCl₅, in halogenierten Lösungsmitteln wie z.B. CH₂Cl₂ oder CHCl₃ bei Temperaturen zwischen -40°C und 80°C zur Reaktion gebracht. Das auf diese Weise erhaltene gegebenenfalls substituierte Dihalo-methoxybenzophenon wird mit gegebenenfalls substituierten Phenolen unter Einwirkung einer Base gegebenenfalls in Kombination mit Phasentransferreagenzien in polaren aprotischen Lösungsmitteln umgesetzt. Bevorzugt wird das gegebenenfalls substituierte Difluormethoxybenzophenon mit gegebenenfalls substituierten Phenolen in Lösungsmittelgemischen wie N,N-Dimethylformamid/Toluol, N,N-Dimethylacetamid/Toluol, Dimethylsulfoxid/Toluol oder N-Methylpyrrolidon/Toluol unter Zusatz von z.B. Alkalicarbonaten, Alkalihydroxiden oder organische Basen wie Pyridin oder Diazabicycloundecen gegebenenfalls in Kombination mit Phasentransferreagenzien bei Temperaturen von 80-200°C innerhalb von 1-48 Stunden umgesetzt. Besonders bevorzugt wird das gegebenenfalls substituierte Difluormethoxybenzophenon mit gegebenenfalls substituierten Phenolen in Lösungsmittelgemischen wie N,N-Dimethylacetamid/Toluol, Dimethylsulfoxid/Toluol oder N-Methylpyrrolidon/Toluol unter Zusatz von K₂CO₃ oder K₂CO₃/18-Krone-6, bei Temperaturen von 100-180°C innerhalb von 3-24 Stunden umgesetzt. Dabei wird das gegebenenfalls substituierte Difluormethoxybenzophenon mit gegebenenfalls substituierten Phenolen wie oben beschrieben für 1-6 Stunden unter Rückfluss gerührt, danach das Toluol/Wassergemisch mit Hilfe eines Wasserabscheiders entfernt und dann die Lösung für weitere 1-16 Stunden bei Temperaturen zwischen 130 und 180°C gerührt. Die Aufreinigung des Produkts erfolgt bevorzugterweise über Säulenchromatographie an Kieselgel (z.B. Kieselgel 60, 0040-0,063 mm, Merck) mit einem Gemisch aus n-Hexan und Essigester als Eluent.

Alternativ zu den oben genannten Bedingungen kann auch das zuvor hergestellte Phenolat direkt mit dem Dihalomethoxybenzophenon zur Reaktion gebracht werden. Dabei erübrigt sich im oben beschriebenen Verfahren der Zusatz von Toluol und der Base.

Zur Freisetzung der phenolischen OH-Gruppe kann die Spaltung des Methylethers beispielsweise mit BBr₃ in Dichlormthan, mit Me₃SiI in Chloroform oder mit wässriger HBr in Essigsäure erfolgen. Bevorzugterweise wird diese Reaktion in einem Gemisch aus wässriger HBr-Lösung und HBr-Lösung in Essigsäure durchgeführt.

Verbindungen der Formel 3a in denen Y z.B. für eine Einfachbindung steht lassen sich prinzipiell wie folgt darstellen: Durch Friedel-Crafts-Reaktion von Toluol mit Halogenalkanen erhält man in bekannter Weise substituierte Aromaten, die durch anschließende Oxidation der Methylgruppe am Aromaten, z.B. mit Kaliumpermanganat, in die entsprechend substituierte Benzoesäure überführt werden. Nach dieser Methode lässt sich z.B. 3,5-Di-tert.-butylbenzoesäure in bekannter Weise (s. DE 32 21 818) durch eine Friedel-Crafts-Reaktion von tert.-Butylchlorid und Toluol und anschließender Oxidation mit Kaliumpermanganat in wässrigem Pyridin darstellen. Die auf diese Weise erhaltene substituierte Benzoesäure kann in bekannter Weise mit Oxalylchlorid oder Thionylchlorid in das entsprechende Säurechlorid überführt werden. Das Säurechlorid wird mit gegebenenfalls substituierten, allerdings in para-Stellung zur Methoxygruppe unsubstituierten, Anisolderivaten, bevorzugt unter Einwirkung von z.B. FeCl₃, AlCl₃, BF₃, ZnCl₂, SnCl₄ oder SbCl₅, in halogenierten Lösungsmitteln wie z.B. CH₂Cl₂ oder CHCl₃ bei Temperaturen zwischen -40°C und 80°C zur Reaktion gebracht. Zur Freisetzung der phenolischen OH-Gruppe kann die Spaltung des Methylethers beispielsweise mit BBr₃ in Dichlormethan, mit Me₃SiI in Chloroform oder mit wässriger HBr in Essigsäure erfolgen. Bevorzugt wird diese Reaktion in einem Gemisch aus wässriger HBr-Lösung und HBr-Lösung in Essigsäure durchgeführt.

Außer den phenolischen Verbindungen der Formel (1), (2) und (3) können noch andere Phenole in Mengen bis zu 50 Mol-% bezogen auf die jeweilige Gesamtmenge an Kettenabbrecher zur Herstellung der Polycarbonate, Polyestercarbonate und Polyester mitverwendet werden.

Gegenstand der vorliegenden Erfindung ist somit auch die Verwendung der phenolischen Verbindungen der Formel (1) gegebenenfalls in Kombination mit anderen Phenolen als Kettenabbrecher zur Herstellung von aromatischen Polycarbonaten, aromatischen Polyestercarbonaten und aromatischen Polyestern, wobei die anderen Phenole in Mengen bis zu 50 Mol-% vorzugsweise bis 25 Mol-% bezogen auf die jeweilige Gesamtmolmenge an Kettenabbrecher eingesetzt werden.

Gegenstand der vorliegenden Erfindung sind somit auch thermoplastische Polycarbonate, thermoplastische Polyestercarbonate und thermoplastische Polyester enthaltend von den phenolischen Verbindungen der Formeln (1), (2) und (3) abgeleitete Endgruppen, beispielhaft aber nicht einschränkend durch die Polymere der Formel (5) dargestellt, 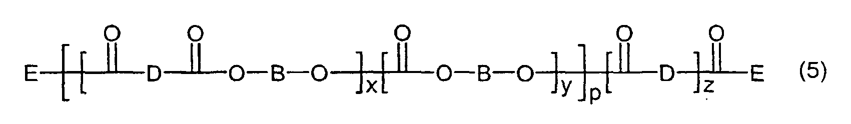 worin 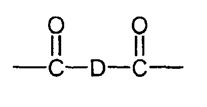 der Rest einer aromatischen Dicarbonsäure ist, -O-B-O- ein Bisphenolatrest ist, "p" eine ganze Zahl zwischen 25 und 700 ist, "x" und "y" Bruchzahlen aus der Reihe 0/p, 1/p, 2/p bis p/p sind, wobei x + y = 1 ist und "z" = Null oder 1 ist und mindestens 50 Mol-% der Reste E in (5) den Phenolatresten entsprechend den phenolischen Verbindungen der Formel (1), (2) und (3) entsprechen und maximal 50 Mol-% der Reste E in (5) einem anderen Phenolatrest als dem entsprechend den phenolischen Verbindungen der Formel (1), (2) oder (3) entsprechen.

Gemäß DE-A 2 119 799 erfolgt die Herstellung von Polycarbonaten unter Beteiligung phenolischer Endgruppen, nach dem Phasengrenzflächenverfahren wie auch dem Verfahren in homogener Phase.

Zur Herstellung von Polycarbonaten nach dem Phasengrenzflächenverfahren sei beispielhaft auf H. Schnell, Chemistry and Physics of Polycarbonates, Polymer Reviews, Vol. 9, Interscience Publishers, New York 1964 S. 33 ff. und auf Polymer Reviews, Vol. 10, "Condensation Polymers by Interfacial and Solution Methods", Paul W. Morgan, Interscience Publishers, New York 1965, Kap. VIII, S. 325 verwiesen.

Daneben ist die Herstellung der erfindungsgemäßen Polycarbonate auch aus Diarylcarbonaten und Diphenolen nach dem bekannten Polycarbonatverfahren in der Schmelze, dem sogenannten Schmelzumesterungsverfahren, möglich, das z.B. in WO-A 01/05866 und WO-A 01/05867 beschrieben ist. Daneben werden Umesterungsverfahren (Acetatverfahren und Phenylesterverfahren) beispielsweise in den US-A 34 94 885, 43 86 186, 46 61 580, 46 80 371 und 46 80 372, in den EP-A 26 120, 26 121, 26 684, 28 030, 39 845, 39 845, 91 602, 97 970, 79 075, 14 68 87, 15 61 03, 23 49 13 und 24 03 01 sowie in den DE-A 14 95 626 und 22 32 977 beschrieben.

Diarylcarbonate im Sinne vorliegender Erfindung sind solche Kohlensäurediester der Formel (6) 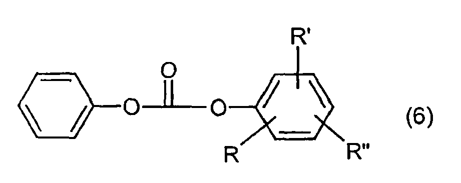 und Formel (7), 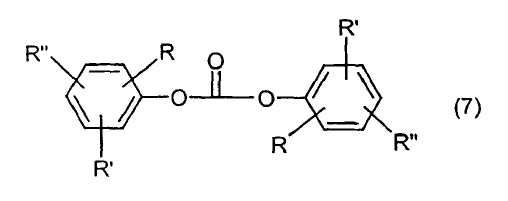 wobei R, R' und R" unabhängig voneinander H, gegebenenfalls verzweigte C₁-C₃₄-Alkyl/Cycloalkyl, C₇-C₃₄-Alkaryl oder C₆-C₃₄-Aryl bzw. C₆-C₃₄-Aryl-oxy darstellen können, beispielsweise
Diphenylcarbonat, Butylphenyl-phenylcarbonat, Di-butylphenylcarbonat, Isobutylphenyl-phenylcarbonat, Di-isobutylphenylcarbonat, tert-Butylphenyl-phenylcarbonat, Di-tert-butylphenylcarbonat, n-Pentylphenyl-phenylcarbonat, Di-(n-pentylphenyl)-carbonat, n-Hexylphenyl-phenylcarbonat, Di-(n-hexylphenyl)carbonat, Cyclohexylphenyl-phenylcarbonat, Di-cyclohexylphenylcarbonat, Phenylphenol-phenylcarbonat, Di-phenylphenolcarbonat, Isooctylphenyl-phenylcarbonat, Di-isooctylphenylcarbonat, n-Nonylphenyl-phenylcarbonat, Di-(n-nonylphenyl)carbonat, Cumylphenyl-phenylcarbonat, Di-cumylphenylcarbonat, Naphthylphenyl-phenylcarbonat, Di-naphthylphenylcarbonat, Di-tert-butylphenyl-phenylcarbonat, Di-(di-tert-butylphenyl)carbonat, Dicumylphenyl-phenylcarbonat, Di-(dicumylphenyl)-carbonat, 4-Phenoxyphenyl-phenylcarbonat, Di-(4-phenoxyphenyl)carbonat, 3-Pentadecylphenyl-phenylcarbonat, Di-(3-pentadecylphenyl)carbonat, Tritylphenylphenylcarbonat, Di-tritylphenylcarbonat,
bevorzugt
Diphenylcarbonat, tert-Butylphenyl-phenylcarbonat, Di-tert-butylphenylcarbonat, Phenylphenol-phenylcarbonat, Di-phenylphenolcarbonat, Cumylphenyl-phenylcarbonat, Di-cumylphenylcarbonat,
besonders bevorzugt Diphenylcarbonat.

Diphenole für die erfindungsgemäßen Polycarbonate können beispielsweise Hydrochinon, Resorcin, Dihydroxybiphenyle, Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulf oxide, α,α'-Bis-(hydroxyphenyl)-diisopropylbenzole, sowie deren kernalkylierte und kernhalogenierte Verbindungen sein und auch α,ω- Bis-(hydroxyphenyl)-polysiloxane.

Bevorzugte Diphenole sind beispielsweise 4,4'-Dihydroxybiphenyl (DOD), 2,2-Bis-(4-hydroxyphenyl)-propan (Bisphenol A), 1,1-Bis-(4-hydroxyphenyl)-3,3,5-trimethylcyclohexan (Bisphenol TMC), 1,1-Bis-(4-hydroxyphenyl)-cyclohexan, 2,4-Bis-(4-hydroxyphenyl)-2-methylbutan, 1,1-Bis-(4-hydroxyphenyl)-1-phenylethan, 1,1-Bis-(4-hydroxyphenyl)-*p*-diisopropylbenzol, 1,3-Bis[2-(4-hydroxyphenyl)-2-propyl]-benzol (Bisphenol M), 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 2,2-Bis-(3,5-dichlor-4-hydroxyphenyl)-propan und 2,2-Bis-(3,5-dibrom-4-hydroxyphenyl)-propan.

Besonders bevorzugte Diphenole sind beispielsweise 2,2-Bis-(4-hydroxyphenyl)-propan (Bisphenol A), 1,3-Bis[2-(4-hydroxyphenyl)-2-propyl]-benzol (Bisphenol M), 2,2-Bis-(3,5-dimethyl-4-hydroxyphenyl)-propan, 1,1-Bis-(4-hydroxyphenyl)-1-phenylethan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Ganz besonders bevorzugt sind 2,2-Bis-(4-hydroxyphenyl)-propan (Bisphenol A), 1,3-Bis[2-(4-hydroxyphenyl)-2-propyl]-benzol (Bisphenol M) und 1,1-Bis-(4-hydroxyphenyl)-3,3,5-trimethylcyclohexan (Bisphenol TMC).

Die Diphenole können sowohl allein als auch im Gemisch miteinander verwendet werden; es sind sowohl Homopolycarbonate als auch Copolycarbonate einbezogen. Die Diphenole sind literaturbekannt oder nach literaturbekannten Verfahren herstellbar (siehe z.B. H. J. Buysch et al., Ullmann's Encyclopedia of Industrial Chemistry, VCH, New York 1991, 5. Ed., Vol. 19, p. 348).

Es können auch geringe Mengen, vorzugsweise Mengen zwischen 0,05 und 2,0 Mol-%, bezogen auf die Mole eingesetzter Diphenole, an tri- oder multifunktionellen Verbindungen, insbesondere solchen mit drei oder mehr als drei phenolischen Hydroxygruppen als sogenannte Verzweiger, mitverwandt werden. Dadurch ergeben sich selbstverständlich Abweichungen von der idealisierten und nur beispielhaft angeführten Formel (5), da es dann zu verzweigenden Strukturen in Abweichung von den angegebenen Strukturen D und B kommt.

Einige der verwendbaren Verbindungen mit drei oder mehr als drei phenolischen Hydroxygruppen sind beispielsweise Phloroglucin, 4,6-Dimethyl-2,4,6-tri-(4-hydroxyphenyl)-hept-2-en, 4.6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2-hydroxy-5'-methylbenzyl)-4-methylphenol, 2-(4-Hydroxyphenyl)-2-(3,4-dihydroxyphenyl)-propan, Hexa-[4-(4-hydroxyphenyl-isopropyl)-phenyl]-orthoterephthalsäureester, Tetra-[4-(4-hydroxyphenyl-isopropyl)-phenoxy]-methan, Tetra-(4-hydroxyphenyl)-methan und 1,4-Bis-(4',4" -dihydroxytriphenyl)-methylbenzol.

Weitere mögliche Verzweiger sind 2,4-Dihydroxybenzoesäure, Trimesinsäure, Cyanurchlorid und 3,3-Bis-(3-methyl-4-hydroxyphenyl)-2-oxo-2,3-dihydroindol.

Die gegebenenfalls mitzuverwendenden 0.05 bis 2 Mol-%, bezogen auf eingesetzte Diphenole, an Verzweigern können entweder mit den Diphenolen selbst und den erfindungsgemäßen Molekulargewichtsreglern in der wässrigen alkalischen Phase vorgelegt werden, oder in einem organischen Lösungsmittel gelöst vor der Phosgenierung zugegeben werden.

Die aromatischen Polycarbonate der vorliegenden Erfindung besitzen Gewichtsmittelmolekulargewichte M_{w} (ermittelt durch Gelpermeationschromatographie und Eichung mit Polystyrolstandard) zwischen 5000 und 200000, vorzugsweise zwischen 10000 und 80000 und besonders bevorzugt zwischen 15000 und 40000.

Die relativen Lösungsviskositäten, liegen entsprechend zwischen 1,10 und 1,60 gemessen in Methylenchlorid (0.5 g Polycarbonat in 100 ml Methylenchlorid bei 23°C).

Erfindungsgemäße Polyestercarbonate sind solche, die aus mindestens einem Diphenol, aus mindestens einer aromatischen Dicarbonsäure und aus Kohlensäure aufgebaut sind.

Geeignete aromatische Dicarbonsäuren sind beispielsweise Orthophthalsäure, Terephthalsäure, Isophthalsäure, *tert*.-Butylisophthalsäure, 3,3`-Diphenyldicarbonsäure, 4,4'-Diphenyletherdicarbonsäure, 4,4'-Diphenylsulfondicarbonsäure, 3,4'-Benzophenondicarbonsäure, 2,2-Bis-(4-carboxyphenyl)-propan, Trimethyl-3-phenylindan-4,5-dicarbonsäure.

Von den aromatischen Dicarbonsäuren werden besonders bevorzugt die Terephthalsäure und/oder Isophthalsäure eingesetzt.

Geeignete Diphenole sind die vorstehend für die Polycarbonatherstellung genannten. Die Kohlensäure kann entweder via Phosgen oder via Diphenylcarbonat in die Polyestercarbonate eingebaut werden, je nach Wahl des Herstellungsverfahrens, also je nachdem, ob Phasengrenzflächenpolykondensation oder Schmelzumesterung zur Polyestercarbonatherstellung verwendet wird.

Entsprechendes gilt für die aromatischen Dicarbonsäuren; sie werden entweder als aromatische Dicarbonsäuredichloride im Zweiphasengrenzflächenverfahren oder als Dicarbonsäurediester im Schmelzumesterungsverfahren eingesetzt.

Die Herstellung der erfindungsgemäßen Polyestercarbonate erfolgt nach bekannten Herstellungsverfahren, also wie bereits erwähnt beispielsweise nach dem Phasengrenzflächenverfahren oder nach dem Schmelzumesterungsverfahren.

Die erfindungsgemäßen Polyestercarbonate können sowohl linear als auch in bekannter Weise verzweigt sein. Die aromatischen Polyestercarbonate der vorliegenden Erfindung haben mittlere Gewichtsmittelmolekulargewichte M_{w} (ermittelt durch Gelpermeationschromatographie mit Polystyroleichung) vorzugsweise zwischen 10000 und 250000.

Das molare Verhältnis von Carbonateinheiten zu aromatischen Dicarboxylateinheiten in den erfindungsgemäßen Polyestercarbonaten liegt vorzugsweise bei 95:5 bis 5:95, besonders bevorzugt zwischen 90:10 bis 10:90, insbesondere bevorzugt zwischen 80:20 und 20:80 und ganz besonders bevorzugt zwischen 60:40 und 40:60 vor.

"z" kann im Falle der erfindungsgemäßen Polyester (5) sowohl 0 als auch 1 sein.

Erfindungsgemäße aromatische Polyester sind solche aus mindestens einem Diphenol und mindestens einer aromatischen Dicarbonsäure.

Geeignete Diphenole und Dicarbonsäuren sind die vorstehend für die Polyestercarbonatherstellung genannten.

Die erfindungsgemäßen aromatischen Polyester werden nach bekannten Herstellungsverfahren (siehe z.B. Kunststoff-Handbuch, Bd. VIII, S. 695 ff, Carl-Hanser-Verlag, München, 1973) hergestellt.

Die erfindungsgemäßen aromatischen Polyester können sowohl linear als auch in bekannter Weise verzweigt sein.

Die erfindungsgemäßen aromatischen Polyester besitzen mittlere Gewichtsmittelmolekulargewichte M_{w} (ermittelt nach der Lichtstreuungsmethode), vorzugsweise zwischen 25000 und 70000; dies entspricht Polymerisationsgraden "p" in Formel (5) von etwa 80 bis 270, wobei "x" = 1, "y" = 0 und z = 1 sind.

Die einzusetzende Menge an erfindungsgemäßen Monophenolen der Formel (1), (2) oder (3) zur Herstellung der erfindungsgemäßen Polycarbonate, Polyestercarbonate oder Polyester liegt zwischen 0,5 Mol-% und 8 Mol-%, vorzugsweise zwischen 2 Mol-% und 6 Mol-% bezogen auf die jeweils eingesetzten Diphenole.

Als weitere Kettenabbrecher sind die üblichen Monophenole wie beispielsweise Phenol, 4-Alkylphenole und 4-Cumylphenol geeignet.

Gegenstand der vorliegenden Erfindung ist somit auch ein Verfahren zur Herstellung der erfindungsgemäßen Polycarbonate, Polyestercarbonate oder Polyester aus Diphenolen, Monophenolen, Kohlensäurederivaten und/oder Dicarbonsäurederivaten nach an sich bekannten Verfahrensbedingungen, das dadurch gekennzeichnet ist, dass man als Kettenabbrecher Monophenole der Formel (1), (2) oder (3) in Mengen von 0.5 Mol-% bis 8 Mol-%, vorzugsweise von 2 Mol-% bis 6 Mol-% , bezogen jeweils auf Mole Diphenole, einsetzt, wobei davon bis zu 50 Mol-%, vorzugsweise bis zu 25 Mol-%, bezogen jeweils auf die Gesamtmenge an Kettenabbrecher, durch andere Monophenole ersetzt werden können.

Im Falle des Phasengrenzflächenpolykondensationsverfahrens können die Kettenabbrecher der Formel (1), (2) oder (3) vor, während oder nach der Phosgenierung in Lösung zugesetzt werden. Die für das Lösen der Kettenabbrecher der Formel (1), (2) oder (3) geeigneten Lösungsmittel sind beispielsweise Methylenchlorid, Chlorbenzol oder Acetonitril sowie Mischungen dieser Lösungsmittel.

Im Falle des Schmelzeumesterungsverfahren besteht nach dem erfindungsgemäßen Verfahren die Möglichkeit, die Kettenabbrecher der Formel (1), (2) oder (3) zu jedem Zeitpunkt der Reaktion zuzugeben; dabei kann die Zugabe in mehrere Portionen aufgeteilt werden.

Gegenstand der Erfindung sind auch die nach dem erfindungsgemäßen Verfahren erhältlichen Polycarbonate, Polyestercarbonate und Polyester.

Diphenole zur Herstellung der erfindungsgemäßen Polycarbonate, Polyestercarbonate und Polyester können auch Polymerisate oder Kondensate mit phenolischen Endgruppen sein, so dass erfindungsgemäß auch Polycarbonate bzw. Polyestercarbonate bzw. Polyester mit Blockstrukturen einbezogen sind.

Die erfindungsgemäßen Polycarbonate, Polyestercarbonate und Polyester können in bekannter Weise aufgearbeitet und zu beliebigen Formkörpern verarbeitet werden, beispielsweise durch Extrusion oder Spritzguss.

Den erfindungsgemäßen Polycarbonaten, Polyestercarbonaten und Polyestern können noch andere aromatische Polycarbonate und/oder andere aromatische Polyestercarbonate und/oder andere aromatische Polyester in bekannter Weise zugemischt werden.

Den erfindungsgemäßen Polycarbonaten, Polyestercarbonaten und Polyestern können noch die für diese Thermoplasten üblichen Additive wie Füllstoffe, UV-Stabilisatoren, Thermostabilisatoren, Antistatika und Pigmente in den üblichen Mengen zugesetzt werden; gegebenenfalls können das Entformungsverhalten, das Fließverhalten, und/oder die Flammwidrigkeit noch durch Zusatz externer Entformungsmittel, Fließmittel, und/oder Flammschutzmittel verbessert werden (z.B. Alkyl- und Arylphosphite, -phosphate, -phosphane, -niedermolekulare Carbonsäureester, Halogenverbindungen, Salze, Kreide, Quarzmehl, Glas- und Kohlenstofffasern, Pigmente und deren Kombination. Solche Verbindungen werden z.B. in WO 99/55772, S. 15 - 25, und in "Plastics Additives", R. Gächter und H. Müller, Hanser Publishers 1983, beschrieben).

Die erfindungsgemäßen Polycarbonate, Polyestercarbonate und Polyester, gegebenenfalls in Abmischung mit anderen Thermoplasten und/oder üblichen Additiven können zu beliebigen Formkörpern/Extrudaten verarbeitet überall dort eingesetzt werden, wo bereits bekannte Polycarbonate, Polyestercarbonate und Polyester eingesetzt werden. Aufgrund ihres Eigenschaftsprofils eignen sie sich insbesondere als Substratmaterialien für optische Datenspeicher wie z.B. CD, CD-R, DVD, oder DVD-R, sind aber auch beispielsweise als Folien im Elektrosektor als Formteile im Fahrzeugbau und als Platten für Abdeckungen im Sicherheitsbereich einsetzbar. Weitere mögliche Anwendungen der erfindungsgemäßen Polycarbonate sind:
1. Sicherheitsscheiben, die bekanntlich in vielen Bereichen von Gebäuden, Fahrzeugen und Flugzeugen erforderlich sind, sowie als Schilde von Helmen.
2. Herstellung von Folien, insbesondere Skifolien.
3. Herstellung von Blaskörpern (siehe beispielsweise US-Patent 2 964 794), beispielsweise 1 bis 5 Gallon Wasserflaschen.
4. Herstellung von lichtdurchlässigen Platten, insbesondere von Hohlkammerplatten, beispielsweise zum Abdecken von Gebäuden wie Bahnhöfen, Gewächshäusem und Beleuchtungsanlagen.
5. Herstellung optischer Datenspeicher.
6. Zur Herstellung von Ampelgehäusen oder Verkehrsschildern.
7. Zur Herstellung von Schaumstoffen (siehe beispielsweise DE-AS 1 031 507).
8. Zur Herstellung von Fäden und Drähten (siehe beispielsweise DE-AS 1 137 167 und DE-OS 1 785 137).
9. Als transluzente Kunststoffe mit einem Gehalt an Glasfasern für lichttechnische Zwecke (siehe beispielsweise DE-OS 1 554 020).
10. Als transluzente Kunststoffe mit einem Gehalt an Bariumsulfat, Titandioxid und oder Zirkoniumoxid bzw. organischen polymeren Acrylatkautschuken (EP-A 634 445, EP-A 269324) zur Herstellung von lichtdurchlässigen und lichtstreunenden Formteilen.
11. Zur Herstellung von Präzisionsspritzgußteilchen, wie beispielsweise Linsenhalterungen. Hierzu verwendet man Polycarbonate mit einem Gehalt an Glasfasern, die gegebenenfalls zusätzlich etwa 1 - 10 Gew.-% MoS₂, bezogen auf Gesamtgewicht, enthalten.
12. Zur Herstellung optischer Geräteteile, insbesondere Linsen für Foto- und Filmkameras (siehe beispielsweise DE-OS 2 701 173).
13. Als Lichtübertragungsträger, insbesondere als Lichtleiterkabel (siehe beispielsweise EP-A1 0 089 801).
14. Als Elektroisolierstoffe für elektrische Leiter und für Steckergehäuse sowie Steckverbinder.
15. Herstellung von Mobiltelefongehäusen mit verbesserter Beständigkeit gegenüber Parfüm, Rasierwasser und Hautschweiß.
16. Network interface devices
17. Als Trägermaterial für organische Fotoleiter.
18. Zur Herstellung von Leuchten, z. B. Scheinwerferlampen, als sogenannte "head-lamps", Streulichtscheiben oder innere Linsen.
19. Für medizinische Anwendungen, z.B. Oxygenatoren, Dialysatoren.
20. Für Lebensmittelanwendungen, wie z. B. Flaschen, Geschirr und Schokoladenformen.
21. Für Anwendungen im Automobilbereich, wo Kontakt zu Kraftstoffen und Schmiermitteln auftreten kann, wie beispielsweise Stoßfänger ggf. in Form geeigneter Blends mit ABS oder geeigneten Kautschuken.
22. Für Sportartikel, wie z. B. Slalomstangen oder Skischuhschnallen.
23. Für Haushaltsartikel, wie z. B. Küchenspülen und Briefkastengehäuse.
24. Für Gehäuse, wie z. B. Elektroverteilerschränke.
25. Gehäuse für Elektrozahnbürsten und Föngehäuse
26. Transparente Waschmaschinen - Bullaugen mit verbesserter Beständigkeit gegenüber der Waschlösung.
27. Schutzbrillen, optische Korrekturbrillen.
28. Lampenabdeckungen für Kücheneinrichtungen mit verbesserter Beständigkeit gegenüber Küchendunst insbesondere Öldämpfen.
29. Verpackungsfolien für Arzneimittel.
30. Chip-Boxen und Chip-Träger
31. Für sonstige Anwendungen, wie z. B. Stallmasttüren oder Tierkäfige.

Die Formkörper und Extrudate aus den erfindungsgemäßen Polymeren sind ebenfalls Gegenstand dieser Anmeldung.

### Beispiele

### Beispiel 1:

### Darstellung von Difluorbenzoesäurechlorid

In einem Rundkolben werden 22,0 g (0.139 mol) 3,5-Difluorbenzoesäure unter Argon vorgelegt und 323,33 g (2,718 mol) Thionylchlorid unter Rühren hinzugetropft. Die Mischung wird nach Zugabe einiger Tropfen von *N,N-*Dimethylformamid auf 70°C erwärmt und bis zum Ende der Gasentwicklung gerührt. Anschließend wird das verbleibende Thionylchlorid im Wasserstrahlvakuum abdestilliert. Man erhält 21,19 g (86%) eines zähflüssigen Öls.
¹H-NMR (400 MHz, CDCl₃) δ = 7,69-7,62 (m, 2 H), 7,19-7,12 (m, 1 H).

### Beispiel 2:

### Darstellung von 3,5-Difluor-4'-methoxybenzophenon

In einem Rundkolben werden 12,98 g (0,120 mol) Anisol in 40 ml Dichlormethan unter Argonatmosphäre gelöst und auf 0°C abgekühlt. Man gibt 16,0 g (0,120 mol) Aluminiumchlorid in kleinen Portionen hinzu. Es wird für 10 Minuten gerührt. Zu diesem Gemisch wird eine Lösung von 21,19 g (0,120 mol) Difluorbenzoesäurechlorid (gelöst in 100 ml Dichlormethan) getropft. Es wird noch 4 Stunden bei 0°C gerührt und lässt die Lösung auf Raumtemperatur erwärmen. Man gibt vorsichtig 90 ml Salzsäure-Lösung (10 %) hinzu und trennt die organische von der wässrigen Phase ab. Die wässrige Phase wird mit Dichlormethan extrahiert. Die vereinigten organischen Phasen werden mit NaOH (1 mol/l) gewaschen und abschließend 1 x mit entmineralisiertem Wasser und 2 x mit gesättigter NaCl-Lösung neutral gewaschen, über Magnesiumsulfat getrocknet, filtriert und eingeengt.

Der auf diese Weise erhaltene Feststoff wird in *n*-Hexan umkristallisiert. Nach Trocknung verbleiben weiße, nadelförmige Kristalle (19,25 g, 65 %; Smp.:90°C).
¹H-NMR (400 MHz, CDCl₃) δ = 7.81 (d, 2H), 7,28-7,24 (m, 2 H), 7,05-6,96 (m 3 H), 3,90 (s, 3 H).

### Beispiel 3:

### Darstellung von 3,5-Diphenyloxy-4'-methoxybenzophenon

19,0 g (0,077 mol) 3,5-Difluor-4'-methoxybenzophenon werden unter Argon in einem Gemisch aus 200 ml Dimethylsulfoxid und 200 ml Toluol gelöst. Zu dieser Lösung werden 28,56 g (0,207 mol) feingepulvertes Kaliumcarbonat gegeben. Die Lösung wird unter Rückfluss für 4 Stunden gerührt, das entstehende Wasser wird dabei am Wasserabscheider entfernt. Anschließend wird das Toluol vollständig abdestilliert und das Reaktionsgemisch noch für 3 Stunden bei 150°C gerührt. Es werden nochmals 28,56 g (0,207 mol) feingepulvertes Kaliumcarbonat und 50 ml Toluol zugegeben. Es wird auf Rückfluss erhitzt und das Toluol abdestilliert. Man lässt die Lösung noch 4 Stunden bei 150°C rühren. Nach Abkühlen wird die Lösung auf Eis gegossen. Durch Zugabe von HCl-Lösung (10 %) wird die Lösung neutralisiert. Man extrahiert mehrmals mit Diethylether. Die vereinigten organischen Phasen werden mit gesättigter Natriumchlorid Lösung gewaschen. Es wird über Magnesiumsulfat getrocknet und das Lösungsmittel im Vakuum entfernt. Das Rohprodukt wird an Kieselgel (Kieselgel 60, 0040-0,063 mm, Merck), mit *n*-Hexan/Ethylacetat (19:1) chromatographiert. Man erhält 14,90 g (49 %) eines hellgelben Öls.
¹H-NMR (400 MHz, CDCl₃) δ = 7,84-7,78 (m, 2 H), 7,39-7,30 (m, 4 H), 7,15-7,09 (m, 2 H), 7,08-7,02 (m, 6 H), 6,95-6,90 (m, 2 H), 6,86 (t, 1 H), 3,86 (s, 3H).
HPLC-MS (ES), m/z: 396 [M⁺].

### Beispiel 4:

### Darstellung von 3,5-Diphenyloxy-4'-hydroxybenzophenon

14,90 g (0.038 mol) 3,5-Diphenyloxy-4'-methoxybenzophenon werden unter Argon in einem Gemisch aus 60 ml Bromwasserstoffsäure (48 % in Wasser) und 120 ml Bromwasserstoffsäure (Lösung, 33 % in Essigsäure) gelöst. Es wird für 4 Stunden unter Rückfluss gerührt. Es werden zusätzlich 100 ml Bromwasserstoffsäure (Lösung, 33 % in Essigsäure) und 50 ml Bromwasserstoffsäure (48 % in Wasser) zur Reaktionslösung gegeben. Man lässt weitere 6 Stunden unter Rückfluss rühren. Das Reaktionsgemisch wird dann auf Eis gegossen und mehrmals mit Diethylether extrahiert. Die organischen Phasen werden vereinigt und mehrfach mit NaOH-Lösung (20 %) extrahiert. Die wässrigen Phasen werden vereinigt und mit HCl-Lösung neutral gewaschen. Die neutralisierte wässrige Phase wird abschließend noch mehrmals mit Diethylether extrahiert. Die organischen Phasen werden vereinigt, über Magnesiumsulfat getrocknet und das Lösungsmittel im Vakuum entfernt. Das Rohprodukt wird in wenig Diethylether aufgenommen und über Kieselgel filtriert. Nach Entfernen des Lösungsmittels und Trocknen des Rückstandes im Hochvakuum erhält man 11.25 g (78 %) eines weißen Feststoffs.
¹H-NMR (400 MHz, CDCl₃) δ = 7,79-7,74 (m, 2 H), 7,38-7,31 (m, 4 H), 7,16-7,10 (m, 2 H), 7,08-7,02 (m, 6 H), 6,89-6,83 (m, 3 H), 5,35 (s, 1 H).

### Beispiel 5:

### Darstellung von 3,5-Bis-(3,5-di-tert.-butylphenyloxy)-4'-methoxybenzophenon

1,35 g (5,4 mmol) 3,5-Difluor-4'-methoxybenzophenon werden in einem Gemisch aus 80 ml Dimethylsulfoxid und 80 ml Toluol unter Argonatmosphäre gelöst. Zu dieser Lösung gibt man 2,03 g (14,7 mmol) feingepulvertes Kaliumcarbonat. Unter Rühren werden 4,49 g (21,8 mmol) 3,5-Di-*tert*.-butylphenol und 0,575 g (2,2 mmol) Hexaoxacyclooctadecan (18-Krone-6) zugegeben. Das Gemisch wird unter Rückfluss für 4 Stunden gerührt und das während der Reaktion entstandene Wasser am Wasserabscheider entfernt. Es werden nochmals 0,575 g (2,2 mmol) Hexaoxacyclooctadecan (18-Krone-6) und 0,98 g (7,1 mmol) feingepulvertes Kaliumcarbonat zum Reaktionsgemisch gegeben. Es wird für weitere 6 Stunden bei 150°C gerührt. Man lässt Abkühlen und gießt die Lösung auf Eis. Durch Zugabe von HCl-Lösung (10 %) wird die Lösung neutralisiert. Es wird mehrfach mit Diethylether extrahiert. Die vereinigten organischen Phasen werden abschließend mit gesättigter NatriumchloridLösung gewaschen. Die organische Phase wird über Magnesiumsulfat getrocknet und das Lösungsmittel im Vakuum entfernt. Das Rohprodukt wird an Kieselgel (Kieselgel 60, 0040-0,063 mm, Merck) mit einem Gemisch aus *n*-Hexan und Ethylacetat (19:1) als Eluent chromatographiert. Man erhält 2.00 g (59 %) als hellgelben Feststoff.
¹H-NMR (400 MHz, CDCl₃) δ = 7,85-7,80 (m, 2 H), 7,19-7,16 (m, 2 H), 7,05-7,01 (m, 2 H), 6,94-6,88 (m, 6 H), 6,81-6,78 (m, 1 H), 3,86 (s, 3 H), 1,28 (s, 36).
MALDI-TOF: 621 [M + H⁺], 627 [M + Li⁺].

### Beispiel 6:

### Darstellung von 3,5-Bis-(3,5-di-tert.-butylphenyloxy)-4'-hydroxybenzophenon

2,80 g (4,5 mmol) 3,5-Bis-(3,5-di-*tert*.-butylphenyloxy)-4'-methoxybenzophenon werden unter Argon in 100 ml Bromwasserstoff-Lösung (33 % in Essigsäure) gelöst. Man gibt 0,23 g (0,5 mmol) Hexadecyltributylphosphoniumbromid hinzu. Die Lösung wird für 3 Stunden bei 50°C gerührt. Der Reaktionsverlauf wird über DC-Chromatographie verfolgt. Es werden zusätzlich 100 ml Bromwasserstoffsäure (33 % in Eisessig) und 20 ml wässrige Bromwasserstoffsäure (48 %) zur Reaktionslösung gegeben. Die Lösung wird weitere 18 Stunden unter Rückfluss gerührt. Nach Abkühlung der Lösung wird das Reaktionsgemisch auf Eis gegossen und mehrfach mit Diethylether extrahiert. Die vereinigten organischen Phasen werden mit gesättigter NaCl-Lösung gewaschen. Man trocknet über Magnesiumsulfat und entfernt das Lösungsmittel im Vakuum. Das Rohprodukt wird an Kieselgel (Kieselgel 60, 0040-0,063 mm, Merck) mit einem Gemisch aus *n*-Hexan/Ethylacetat (9:1) chromatographiert. Nach Entfernen des Lösungsmittels und Trocknen des Rückstandes im Hochvakuum erhält man 2,47 g (90 %) eines weißen Feststoffs.
¹H-NMR (400 MHz, CDCl₃) δ = 7,79-7,77 (m, 2 H), 7,18 (s, 2 H), 7,03 (s, 2 H), 6,89 (s, 4 H), 6,87-6,85 (m, 2 H), 6,79 (s, 1 H), 1,28 (s, 36 H).

### Beispiel 7:

### Darstellung von 3,5-Di-tert.-butyl-4'-methoxybenzophenon

5,00 g (0,021 mol) Di-*tert*.-butylbenzoesäure werden unter Argon vorgelegt; 49,69 g (0,418 mol) Thionylchlorid werden unter Rühren hinzugetropft. Zur Reaktionslösung werden noch 0,05 ml *N*,*N*-Dimethylformamid gegeben. Die Mischung wird unter Rühren auf 70°C erwärmt und gerührt bis die Gasentwicklung beendet ist. Danach wird das überschüssige Thionylchlorid abdestilliert. Es bleiben 5,39 g eines farblosen viskosen Öls zurück, welches ohne weitere Aufarbeitung eingesetzt wird.

In einem Rundkolben werden 2,25 g (0,021 mol) Anisol in 30 ml Methylenchlorid gelöst und auf 0°C abgekühlt. Zu dieser Lösung werden in kleinen Portionen 2,78 g (0,021 mol) Aluminiumchlorid zugegeben und rührt anschließend für 10 Minuten. Das Säurechlorid (farbloses Öl, s.o.) wird in 50 ml Methylenchlorid gelöst und zur Lösung aus Anisol/AlCl₃ bei 0°C hinzugetropft. Das Reaktionsgemisch wird nach vollständiger Zugabe des Säurechlorids noch für 75 Minuten bei 0°C gerührt. Danach wird die Lösung auf Raumtemperatur erwärmt.

22 ml HCl-Lösung (10 %) werden vorsichtig zum Reaktionsgemisch zugetropft. In einem Scheidetrichter werden die Phasen getrennt. Die wässrige Phase wird mit Methylenchlorid extrahiert und die organischen Phasen vereinigt. Diese wird mit dest. Wasser und abschließend mit gesättigter NaCl-Lösung gewaschen. Man trocknet über Magnesiumsulfat und entfernt das Lösungsmittel im Vakuum. Nach Trocknung im Hochvakuum erhält man 6,38 g (94 %) in Form eines hellgelben Feststoffs.
¹H-NMR (400 MHz, CDCl₃) δ = 7,85 (d, 2 H), 7,62 (s, 1 H), 7,59 (s, 2 H), 6,97 (d, 2 H), 3,90 (s, 3 H), 1,36 (s, 18 H).

### Beispiel 8:

### Darstellung von 3,5-Di-tert.-butyl-4'-hydroxybenzophenon

14,70 g (0.045 mol) 3,5-Di-*tert*.-butyl-4'-methoxybenzophenon werden unter Argonatmosphäre in 200 ml Bromwasserstoffsäure (33 % Lösung in Eisessig) gelöst. Es werden 2.30 g (0,004 mol) Hexadecyltributylphosphoniumbromid und 100 ml Bromwasserstoffsäure (48 % Lösung in Wasser) hinzugegeben und die Lösung für 2 Stunden unter Rückfluss erhitzt. Es werden nochmals 50 ml Bromwasserstoffsäure (33 % Lösung in Eisessig) und 25 ml Bromwasserstoffsäure (48 % Lösung in Wasser) hinzugegeben. Die Lösung wird weitere 2 Stunden unter Rückfluss gerührt.

Nach Abkühlen werden vorsichtig 300 ml Eiswasser zum Reaktionsgemisch gegeben. Es wird 3 x mit Diethylether extrahiert. Die vereinigten organischen Phasen werden mit gesättigter Nacl-Lösung gewaschen und über Magnesiumsulfat getrocknet. Das Lösungsmittel wird im Vakuum entfernt. Das Rohprodukt wird in einer Mischung aus 200 ml *n*-Hexan und 30 ml Chloroform umkristallisiert. Man erhält einen hellbraunen Rückstand. Die Mutterlauge wird eingeengt und der verbleibende Rückstand nochmals in einem Gemisch aus 80 ml *n*-Hexan und 5 ml Chloroform umkristallisiert. Die gereinigten Produktfraktionen werden vereinigt. Man erhält 7,50 g (53 %) in Form eines hellbraunen Feststoffs.
¹H-NMR (400 MHz, CDCl₃) δ = 7,79 (d, 2 H), 7,63 (s, 1 H), 7,59 (s, 2 H), 6,93 (d, 2 H), 6,46 (s, 1 H), 1,34 (s, 18 H).

### Beispiel 9:

### Darstellung von Polycarbonat mit verzweigter Endgruppe

In einem Kolben werden bei Raumtemperatur 8,56 g (0,038 mol) 2,2-Bis-(4-hydroxyphenyl)-propan und 3,30 g NaOH (220 Mol-%, bezogen auf Bisphenol-Komponente) in 135 ml Wasser unter Stickstoffatmosphäre gelöst. Zu diesem Gemisch werden 0,86 g (2,3 mmol) 3,5-Diphenyloxy-4'-hydroxybenzophenon (s. Beispiel 4) gelöst in 135 ml Dichlormethan gegeben. Man lässt für 5 Minuten Rühren. Bei Raumtemperatur (25°C) und unter kräftigem Rühren werden 7,37 g (200 Mol-%, bezogen auf Bisphenol-Komponente) Phosgen eingeleitet. Der pH-Wert wird dabei durch Nachdosieren von 40%iger NaOH-Lösung im Bereich von pH = 12,5-13 gehalten. Nach beendeter Einleitung wird die Apparatur 5 Minuten mit Stickstoff gespült. Man gibt zum Reaktionsgemisch 0,042 g (1 Mol-%) *N-*Ethylpiperidin gelöst in 5 ml Dichlormethan. Man lässt noch 45 Minuten Rühren. Daraufhin wird mit Dichlormethan verdünnt und die organische Phase abgetrennt. Nach Extraktion der organischen Phase mit gleichem Volumen an 10 %iger Salzsäure wird die organische Phase abgetrennt und noch 5 x mit Wasser elektrolytfrei gewaschen. Das in der organischen Phase gelöste Polymer wird in Methanol gefällt und im Vakuum getrocknet.
Ausbeute: 9,0 g (vor Fällung)
Mₙ = 8833 g/mol
M_{w} = 19059 g/mol
D = 2,16
Tg = 142°C

### Beispiel 10:

### Vergleichsbeispiel

Die Ausführung des Versuchs entspricht der Vorschrift von Beispiel 9. Abweichend wird als Kettenabbrecher statt Diphenyloxy-4'-hydroxybenzophenon 6 Mol-% (bezogen auf Bisphenol-Komponente) *tert*.-Butylphenol eingesetzt.
Mₙ = 10238
M_{w} = 19431
D = 1.90
T_{g} -148°C

### Beispiel 11:

### Darstellung eines Co-Polycarbonats im Schmelzeumesterungsverfahren mit verzweigter Endgruppe

In einem 500 ml Dreihalskolben mit Rührer, Innenthermometer und Vigreuxkolonne (30 cm, verspiegelt) mit Brücke werden 31,96 g (0,14 mol) Bisphenol A, 11,16 g (0,06 mol) Dihydroxybiphenyl, 47,77 g (0,223 mol) Diphenylcarbonat, 0,76 g (0,002 mol) 3,5-Diphenyloxy-4'-hydroxybenzophenon (s. Beispiel 6) und 0,0691 g (8 x 10⁻⁴ Mol-% einer 5 %igen phenolischen Lösung) von Tetraphenylphosphoniumphenolat eingewogen. Die Apparatur wird durch Anlegen von Vakuum und Spülen mit Stickstoff (dreimal) vom Luftsauerstoff befreit und das Gemisch bei 150°C und 100 mbar aufgeschmolzen. Die Temperatur wird auf 190°C erhöht und das entstehende Phenol über 30 Minuten abdestilliert. Nun wird die Temperatur auf 235°C erhöht und 10 Minuten das entstehende Phenol abdestilliert. Dann wird innerhalb von 10 Minuten das Vakuum auf 60 mbar und gleichzeitig die Temperatur auf 300°C eingestellt. Nach wiederum 10 Minuten wird das Vakuum auf 0,5 mbar reduziert und weitere 30 Minuten gerührt. Man erhält ein transparentes Polymer mit einer relativen Viskosität von 1,264.

### Beispiel 12:

### Vergleichsbeispiel zu Beispiel 11

In einem 500 ml Dreihalskolben mit Rührer, Innenthermometer und Vigreuxkolonne (30 cm, verspiegelt) mit Brücke werden 31,96 g (0,14 mol) Bisphenol A, 11,16 g (0,06 mol) Dihydroxybiphenyl, 45,84 g (0,214 mol) Diphenylcarbonat und 0,0691 g (8 x 10⁻⁴ mol % einer 5 %igen phenolischen Lösung) von Tetraphenylphosphoniumphenolat eingewogen. Die Apparatur wird durch Anlegen von Vakuum und Spülen mit Stickstoff (dreimal) vom Luftsauerstoff befreit und das Gemisch bei 150°C und 100 mbar aufgeschmolzen. Die Temperatur wird auf 190°C erhöht und das entstehende Phenol über 30 Minuten abdestilliert. Nun wird die Temperatur auf 235°C erhöht und 10 Minuten das entstehende Phenol abdestilliert. Dann wird innerhalb von 10 Minuten das Vakuum auf 60 mbar und gleichzeitig die Temperatur auf 300°C eingestellt. Nach wiederum 10 Minuten wird das Vakuum auf 0,5 mbar reduziert und weitere 30 Minuten gerührt. Man erhält ein transparentes Polymer mit einer relativen Viskosität von 1,275.

Analog zu Beispiel 9 wurden mit den Kettenabbrechern 3,5-Bis-(3,5-di-*tert*.-butylphenyloxy)-4'-hydroxybenzophenon und 3,5-Di-*tert*.-butyl-4'-hydroxybenzophenon Polycarbonate hergestellt (auf eine Fällung des Polymers in Methanol wurde verzichtet). Diese modifizierten Polycarbonate weisen eine niedrigere Nullviskosität auf als Polycarbonate entsprechender Molmasse, welche *tert*.-Butylphenol als Endgruppe tragen.

| Bsp. -Nr.: | Eingesetzte Endgruppe: | Nullviskosität (Pa.s) (bei 270 °C) | Molgewicht (g/mol) | Glastemperatur (°C) |
|---|---|---|---|---|
| 9 | 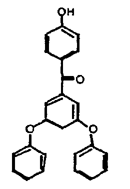 | 200 | M_{w} = 19059 Mₙ = 8833 | 142 |
| 10 | 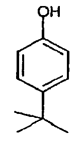 | 300 | M_{w} = 19431 Mₙ = 10238 | 148 |
| 11 | 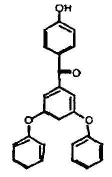 | 420 | M_{w} = 21229 Mₙ = 9547 | 150,5 |
| 12 | 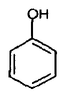 | 500 | M_{w} = 22528 Mₙ = 10602 | 151 |

Anhand vorstehend gezeigter Tabelle wird ersichtlich, dass die erfindungsgemäßen Polycarbonate im Vergleich zu Polycarbonaten mit herkömmlichen Endgruppen wie *tert*.-Butylphenol bzw. Phenol bei annähernd identischem Molekulargewicht überraschenderweise eine reduzierte Nullviskosität aufweisen. Dies gilt sowohl für Polycarbonate die im Phasengrenzflächen- als auch für Polycarbonate die im Schmelzeumesterungsverfahren hergestellt worden sind.

## Patentansprüche

1. Verwendung von Verbindungen der Formel (2) 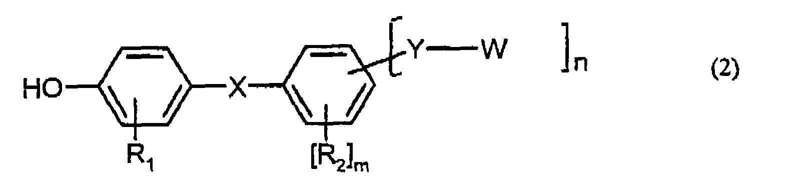 worin
R₁ für H, lineares oder verzweigtes C₁-C₁₈ Alkyl-, Cl, oder Br,
X für eine Einfachbindung oder -O- oder -CO- ,
R₂ für H, lineares oder verzweigtes C₁-C₁₈ Alkyl-, Cl, oder Br,
Y für eine Einfachbindung oder -O- oder -CO-,
W für R⁶ oder einen Rest der Formel 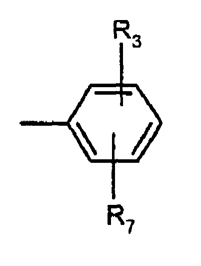
wobei
R⁶ für lineares oder verzweigtes C₁-C₁₈-Alkyl oder cyclisches C₅-C₁₈-Alkyl steht,
R₃, R₇ unabhängig voneinander für H, lineares oder verzweigtes C₁-C₁₈ Alkyl-, cyclisches C₅-C₁₈ Alkyl-, Phenyl-, Phenyloxy-, Phenylcarboxy-, Benzyl-, Benzyloxy-, Naphthyl- oder Naphthyloxy-, Naphthylcarboxyreste stehen,
m für eine Zahl zwischen 0 und 3 und
n für eine Zahl zwischen 2 und 5 stehen,
wobei m und n in Summe ≤ 5 ergibt,
als Kettenabbrecher in der Polycarbonat - Polyester-carbonat - oder Polyestersynthese

2. Verfahren zur Herstellung von Polycarbonat, Polyestercarbonat oder Polyestern, **dadurch gekennzeichnet, dass** als Kettenabbrecher Verbindungen der Formel (2) gemäß Anspruch 1 eingesetzt werden.

3. Polycarbonate, Polyester und Polyestercarbonate, **dadurch gekennzeichnet, dass** sie Endgruppen der Formel 4 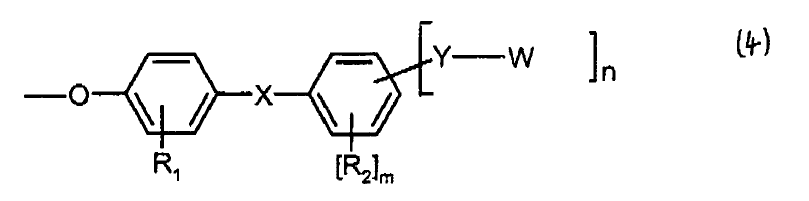 worin
R₁ für H, lineares oder verzweigtes C₁-C₁₈ Alkyl-, Cl, oder Br,
X für eine Einfachbindung oder -O-, oder -CO-,
R₂ für H, lineares oder verzweigtes C₁-C₁₈ Alkyl-, Cl, oder Br,
Y für eine Einfachbindung oder -O-, oder -CO-,
W für R⁶ oder einen Rest der Formel 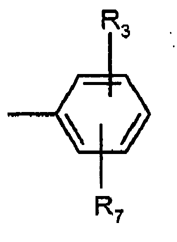
wobei
R⁶ für lineares oder verzweigtes C₁-C₁₈-Alkyl oder cyclisches C₅-C₁₈-Al-kyl steht,
R₃, R₇ unabhängig voneinander für H, lineares oder verzweigtes C₁-C₁₈ Al-kyl-, cyclisches C₅-C₁₈ Alkyl-, Phenyl-, Phenyloxy-, Phenylcarboxy-, Benzyl-, Benzyloxy-, Naphthyl- oder Naphthyloxy-, Naphthylcarboxy-reste stehen,
m für eine Zahl zwischen 0 und 3 und
n für eine Zahl zwischen 2 und 5 stehen,

4. Formkörper und Extrudate aus Polycarbonaten, Polyester oder Polyestercarbonaten gemäß Anspruch 3.

## Claims

1. Use of compounds of the formula (2) 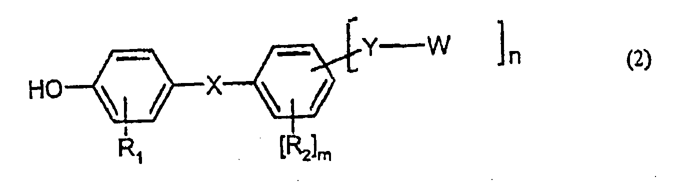 wherein
R₁ denotes H, linear or branched C₁-C₁₈-alkyl, Cl or Br,
X denotes a single bond or -O- or -CO-,
R₂ denotes H, linear or branched C₁-C₁₈-alkyl, Cl or Br,
Y denotes a single bond or -O- or -CO-,
W denotes R⁶ or a radical of the formula 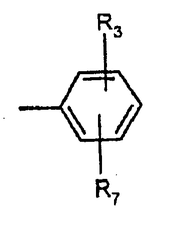
wherein
R⁶ denotes linear or branched C₁-C₁₈-alkyl or cyclic C₅-C₁₈-alkyl,
R₃, R₇ independently of one another denote H, linear or branched C₁-C₁₈-alkyl, cyclic C₅-C₁₈-alkyl, phenyl, phenyloxy, phenylcarboxy, benzyl, benzyloxy, naphthyl or naphthyloxy, naphthylcarboxy radicals,
m denotes a number between 0 and 3, and
n denotes a number between 2 and 5,
the sum total of m and n being ≤ 5,
as chain terminators in the polycarbonate, polyester carbonate or polyester synthesis.

2. Process for the production of polycarbonate, polyester carbonate or polyesters, **characterised in that** as chain terminators compounds of the formula (2) according to claim 1 are used.

3. Polycarbonates, polyesters and polyester carbonates, **characterised in that** they contain terminal groups of the formula (4) 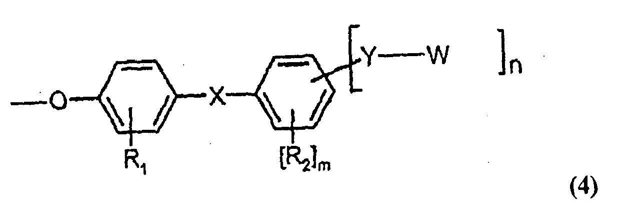 wherein
R₁ denotes H, linear or branched C₁-C₁₈-alkyl, Cl, or Br,
X denotes a single bond or -O- or -CO-,
R₂ denotes H, linear or branched C₁-C₁₈-alkyl, Cl or Br,
Y denotes a single bond or -O- or -CO-,
W denotes R⁶ or a radical of the formula 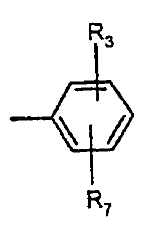
wherein
R⁶ denotes linear or branched C₁-C₁₈-alkyl or cyclic C₅-C₁₈-alkyl,
R₃, R₇ independently of one another denote H, linear or branched C₁-C₁₈-alkyl, cyclic C₅-C₁₈-alkyl, phenyl, phenyloxy, phenylcarboxy, benzyl, benzyloxy, naphthyl or naphthyloxy, naphthylcarboxy radicals,
m denotes a number between 0 and 3, and
n denotes a number between 2 and 5,
the sum total of m and n being ≤ 5.

4. Moulded articles and extrudates of polycarbonates, polyesters or polyester carbonates according to claim 3.

## Revendications

1. Utilisation de composés de la formule (2) : 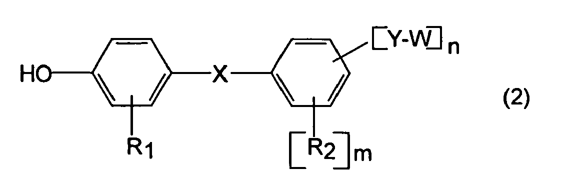 dans laquelle :
R₁ représente H, un radical alcoyle en C₁-C₁₈, linéaire ou ramifié, C1 ou Br,
X représente une simple liaison ou -O- ou -CO-,
R₂ représente H, un radical alcoyle en C₁-C₁₈, linéaire ou ramifié, Cl ou Br,
Y représente une simple liaison ou -O- ou -CO-,
W représente R⁶ ou un reste de la formule : 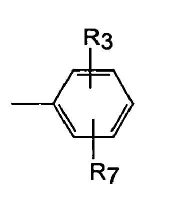
R⁶ représente un radical alcoyle en C₁-C₁₈, linéaire ou ramifié, ou un radical en C₅-C₁₈ cyclique,
R₃, R₇ représentent indépendamment l'un de l'autre, H, un radical alcoyle en C₁-C₁₈, linéaire ou ramifié, un radical en C₅-C₁₈ cyclique, un radical phényle, phényloxy, phénylcarboxy, benzyle, benzyloxy, naphtyle ou naphtyloxy, naphtylcarboxy,
m représente un nombre allant de 0 à 3, et
n représente un nombre allant de 2 à 5,
où m et n donnent une somme ≤ 5,
comme agent d'interruption de chaîne dans la synthèse de polycarbonates, polyester-carbonates ou polyesters.

2. Procédé de préparation de polycarbonates, polyester-carbonates ou polyesters, **caractérisé en ce que** l'on met en oeuvre comme agent d'interruption de chaîne, les composés de la formule (2) selon la revendication 1.

3. Polycarbonates, polyesters et polyester-carbonates, **caractérisés en ce qu'**ils présentent des groupes terminaux de la formule 4 _{:} 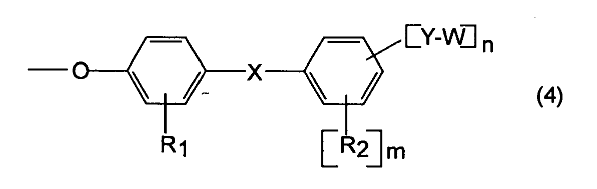 dans laquelle :
R₁ représente H, un radical alcoyle en C₁-C₁₈, linéaire ou ramifié, C1 ou Br,
X représente une simple liaison ou -O- ou -CO-,
R₂ représente H, un radical alcoyle en C₁-C₁₈, linéaire ou ramifié, C1 ou Br,
Y représente une simple liaison ou -O- ou -CO-,
W représente R⁶ ou un reste de la formule : 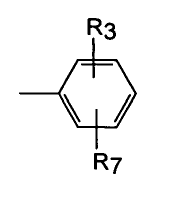
R⁶ représente un radical alcoyle en C₁-C₁₈, linéaire ou ramifié, ou un radical en C₅-C₁₈ cyclique,
R₃, R₇ représentent indépendamment l'un de l'autre, H, un radical alcoyle en C₁-C₁₈, linéaire ou ramifié, un radical en C₅-C₁₈ cyclique, un radical phényle, phényloxy, phénylcarboxy, benzyle, benzyloxy, naphtyle ou naphtyloxy, naphtylcarboxy,
m représente un nombre allant de 0 à 3, et
n représente un nombre allant de 2 à 5,
où m et n donnent une somme ≤ 5.

4. Corps moulés et extrudats des polycarbonates, polyesters ou polyester-carbonates selon la revendication 3.
